# DEMANDE DE BREVET EUROPEEN

(11) **EP 1 036 559 A1**
(43) Date de publication de la demande: **20.09.2000**
(21) Numéro de dépôt: 00400097.2
(22) Date de dépôt: 14.01.2000
(51) Int. Cl.: A61K 7/48, A61K 7/06

(54) **Composition sous forme d'émulsion huile-dans-eau contenant un terpolymère acrylique et ses utilisations notamment cosmétiques.**

(30) Priorité: 17.03.1999 FR 9903320
(71) Demandeur: L'OREAL, 75008 Paris (FR)
(72) Inventeur: Simon, Pascal, 94400 Vitry s/seine (FR); Bordeaux, Dominique, 91310 Longpont S/Orge (FR)
(74) Mandataire: Rasson, Catherine

(57) **Abrégé**

La présente demande se rapporte à une composition sous forme d'émulsion huile-dans-eau, comprenant dans un milieu physiologiquement acceptable, une phase huileuse dispersée dans une phase aqueuse et contenant au moins un terpolymère acrylique obtenu à partir d'un acide carboxylique à insaturation α,β-éthylénique, d'un monomère à insaturation éthylénique non tensioactif différent de (a), et d'un monomère uréthanne non-ionique qui est le produit de réaction d'un composé amphiphile non-ionique monohydrique avec un isocyanate à insaturation monoéthylénique, le rapport en poids terpolymère/phase huileuse allant de 1/50 à 1/125.

La composition obtenue est avantageusement exempte de tout tensioactif classiquement utilisé dans les émulsions H/E. Elle a une très bonne stabilité, quelle que soit la teneur en phase huileuse. Elle peut avoir notamment un usage cosmétique et/ou dermatologique.

L'invention se rapporte aussi à l'utilisation de la dite composition, notamment pour le soin, le traitement ou le nettoyage de la peau, des lèvres et/ou des cheveux, plus particulièrement pour le soin des peaux sèches et/ou des lèvres sèches.

## Description

La présente demande se rapporte à une composition sous forme d'émulsion huile-dans-eau contenant au moins un terpolymère acrylique, et à l'utilisation de la dite composition, en particulier pour le soin et/ou le traitement de la peau du corps ou du visage, des cheveux et/ou des lèvres, et spécialement pour le soin des peaux sèches et/ou des lèvres sèches.

Pour diverses raisons liées en particulier à un meilleur confort d'utilisation (douceur, émollience et autres), les compositions cosmétiques actuelles se présentent le plus souvent sous la forme d'une émulsion du type huile-dans-eau (H/E) constituée d'une phase continue dispersante aqueuse et d'une phase discontinue dispersée huileuse ou d'une émulsion du type eau-dans-huile (E/H) constituée d'une phase continue dispersante huileuse et d'une phase discontinue dispersée aqueuse. Les émulsions H/E sont les plus demandées dans le domaine cosmétique du fait qu'elles comportent comme une phase externe, une phase aqueuse, ce qui leur confère, lors de l'application sur la peau, un toucher plus frais, moins gras et plus léger que les émulsions E/H.

Les émulsions sont généralement stabilisées par des tensioactifs émulsionnants appropriés qui, grâce à leur structure amphiphile, se placent à l'interface huile/eau et stabilisent ainsi les gouttelettes dispersées. Ces émulsionnants présentent cependant l'inconvénient d'être pénétrants et potentiellement irritants pour la peau, les yeux et le cuir chevelu, notamment pour les sujets à peau sensible.

En outre, de telles émulsions peuvent avoir des propriétés cosmétiques et physico-chimiques insuffisantes (toucher huileux, instabilité dans le temps). Le fait d'augmenter le taux des tensioactifs ne résout pas généralement les problèmes mentionnés. La stabilité requise n'est pas toujours atteinte et les propriétés cosmétiques ne sont pas améliorées (toucher cireux, lourd, manque de fraîcheur à l'application). Par ailleurs, comme indiqué ci-dessus, il est aussi déconseillé d'utiliser un trop fort taux de tensioactif pour des raisons d'innocuité.

Une solution pour s'affranchir des phénomènes d'instabilité des émulsions H/E (crémage et déphasage) consiste à ajouter dans l'émulsion des agents épaississants dont la fonction est de créer, au sein de la phase aqueuse, une matrice gélifiée servant à figer les gouttelettes huileuses et assurant un maintien mécanique de l'ensemble de l'émulsion. Toutefois, cette solution présente l'inconvénient de ne pas permettre d'obtenir toutes les textures désirées et en particulier les textures fluides, légères, s'appliquant facilement et rapidement sur la peau sans laisser de film résiduel.

Par ailleurs, il a été envisagé de remplacer les tensioactifs par des polymères comportant dans leur chaîne une partie hydrophile et une partie hydrophobe constituée d'une chaîne grasse, tels que les copolymères d'alkyl-C₁₀-C₃₀-acrylate et d'acide acrylique ou méthacrylique, comme le produit "PEMULEN TR2" commercialisé par la société Goodrich. Cependant, ces polymères présentent l'inconvénient de ne pas permettre l'obtention de composition qui reste stable pendant une grande période de temps quand la quantité d'huile est trop importante ou quand l'émulsion est très fluide, c'est-à-dire dans le cas où l'on cherche à minimiser le taux de polymère pour ne pas avoir une émulsion épaisse.

L'objectif de l'invention est de pouvoir réaliser des émulsions huile-dans-eau stables ne contenant éventuellement pas de tensioactif émulsionnant classiquement utilisé dans les émulsions H/E, présentant de bonnes propriétés cosmétiques sans avoir les inconvénients de l'art antérieur, et ce quelle que soit la quantité d'huile contenue dans l'émulsion.

La demanderesse a découvert de façon inattendue une nouvelle famille de polymères permettant de réaliser des émulsions huile-dans-eau stables bien qu'éventuellement exemptes de tensioactif classiquement utilisé.

La présente invention concerne une composition sous forme d'émulsion huile-dans-eau comprenant dans un milieu physiologiquement acceptable, une phase huileuse dispersée dans une phase aqueuse, caractérisée par le fait qu'elle contient au moins un terpolymère acrylique obtenu à partir de (a) un acide carboxylique à insaturation α,β-éthylénique, (b) un monomère à insaturation éthylénique non tensioactif différent de (a), et (c) un monomère uréthanne non-ionique qui est le produit de réaction d'un composé amphiphile non-ionique monohydrique avec un isocyanate à insaturation monoéthylénique, le rapport en poids terpolymère/phase huileuse allant de 1/50 à 1/125.

La composition obtenue a une texture homogène et agréable à l'application. En outre, le terpolymère acrylique utilisé dans la composition selon l'invention permet de préparer des émulsions huile-dans-eau, qui restent stables dans le temps à température ambiante ou à des températures plus élevées et de conserver ces propriétés de l'émulsion, quelle que soit la fluidité de l'émulsion et sa teneur en phase huileuse. On peut donc ainsi préparer aussi bien des émulsions épaisses ayant une teneur élevée en phase huileuse, particulièrement efficaces pour le traitement des peaux sèches que des émulsions très fluides. La viscosité des émulsions peut donc varier dans une large mesure et aller notamment de 0,05 Pa.s à 8 Pa.s, ces viscosités étant mesurées à environ 25°C à l'aide du viscosimètre "Rhéomat 180" qui est, de manière générale, équipé d'un mobile 2 pour les gammes de viscosités de 0,02 Pa.s à 0,7 Pa.s, d'un mobile 3 pour les gammes de viscosités de 0,2 Pa.s à 4 Pa.s, et d'un mobile 4 pour les gammes de viscosités de 2 Pa.s à 23 Pa.s.

Avantageusement, l'émulsion de l'invention est exempte de tensioactif classiquement utilisé dans les H/E et elle présente de ce fait l'avantage de ne pas être irritante pour les peaux particulièrement sensibles. En outre, cette émulsion présente l'avantage de permettre l'incorporation d'actifs thermosensibles car elle peut être fabriquée à température ambiante, à la fois dans l'étape de neutralisation du polymère et dans l'étape de dispersion de la phase huileuse.

Le terpolymère acrylique utilisé conformément à l'invention est soluble ou gonflable dans les alcalins. Il est de préférence caractérisé par le fait qu'il comprend, par rapport au poids total du terpolymère, :
(a) environ 20 à 70 % en poids, de préférence 25 à 55 % en poids, d'un acide carboxylique à insaturation α,β-monoéthylénique ;
(b) environ 20 à 80 % en poids, de préférence 30 à 65 % en poids, d'un monomère à insaturation monoéthylénique non tensioactif différent de (a) ; et
(c) environ 0,5 à 60 % en poids, de préférence 10 à 50 % en poids, d'un monomère uréthane non-ionique qui est le produit de réaction d'un composé amphiphile non-ionique monohydrique avec un isocyanate à insaturation monoéthylénique.

L'acide carboxylique à insaturation α,β-monoéthylénique (a) peut être choisi parmi de nombreux acides et en particulier l'acide acrylique, l'acide méthacrylique, l'acide crotonique, l'acide itaconique et l'acide maléique. Il s'agit de préférence de l'acide méthacrylique. Une large proportion d'acide est préférable pour donner une structure polymère qui se solubilise et donne un épaississant par réaction avec un composé alcalin comme l'hydroxyde de sodium, les alcanolamines, l'aminométhyl-propanol, ou l'aminométhyl-propanediol.

Le terpolymère contient un monomère (b) à insaturation monoéthylénique qui n'a pas de propriété tensioactive et qui est présent aussi de préférence en une proportion importante telle qu'indiquée ci-dessus. Les monomères préférés sont ceux qui donnent des polymères insolubles dans l'eau lorsqu'ils sont homopolymérisés et ils sont illustrés par les acrylates et méthacrylates d'alkyle en C₁-C₄ comme l'acrylate de méthyle, l'acrylate d'éthyle, l'acrylate de butyle ou les méthacrylates correspondants. Les monomères plus particulièrement préférés sont l'acrylate de méthyle et l'acrylate d'éthyle. D'autres monomères pouvant être utilisés sont le styrène, le vinyltoluène, l'acétate de vinyle, l'acrylonitrile et le chlorure de vinylidène. Les monomères non réactifs sont préférés, ces monomères étant ceux dans lesquels le groupe éthylénique unique est le seul groupe réactif dans les conditions de la polymérisation. Toutefois, des monomères qui contiennent des groupes réactifs sous l'action de la chaleur comme l'acrylate d'hydroxyéthyle peuvent éventuellement être utilisés.

Les composés amphiphiles non-ioniques monohydriques utilisés pour obtenir le monomère uréthane non-ionique (c) sont bien connus et sont généralement des composés hydrophobes alcoxylés contenant un oxyde d'alkylène formant la partie hydrophile de la molécule. Les composés hydrophobes sont généralement constitués par un alcool aliphatique ou un alkylphénol dans lesquels une chaîne carbonée contenant au moins six atomes de carbone constitue la partie hydrophobe du composé amphiphile.

Les composés amphiphiles non-ioniques monohydriques préférés sont des composés ayant la formule (I) suivante :

R-(OCH₂CHR')ₘ-(OCH₂CH₂)ₙ-OH (I)

dans laquelle R est choisi parmi les groupes alkyle comportant de 6 à 30 atomes de carbone et les groupes aralkyle ayant des radicaux alkyle comportant de 8 à 30 atomes de carbone, R' est choisi parmi les groupes alkyle comportant de 1 à 4 atomes de carbone, n est un nombre moyen allant d'environ 6 à 150 et m est un nombre moyen allant d'environ 0 à 50, à la condition que n soit au moins aussi grand que m et que n + m = 6 à 150.

De préférence, dans les composés de formule (I), le groupe R est choisi parmi les groupes alkyle comportant de 18 à 26 atomes de carbone et les groupes alkyl-(C₈-C₁₃)-phényle ; le groupe R' est le groupe méthyle ; m = 0 et n = 6 à 150. Le composé de formule (I) peut en particulier être un dérivé oxyalkyléné, en particulier oxyéthyléné, d'un alcool aliphatique d'origine végétale et notamment de l'alcool béhénylique, le radical R dans la formule (I) étant alors le radical béhényle.

L'isocyanate à insaturation monoéthylénique utilisé pour former le monomère uréthane non-ionique (c) peut être choisi parmi des composés très variés. On peut utiliser tout composé contenant une insaturation copolymérisable telle qu'une insaturation acrylique, méthacrylique ou allylique. L'isocyanate à insaturation monoéthylénique préféré est l'α,α-diméthyl-m-isopropényl-benzyl-isocyanate.

Le terpolymère acrylique défini ci-dessus est obtenu par copolymérisation en dispersion aqueuse des composants (a), (b) et (c), copolymérisation qui est tout à fait usuelle et décrite notamment dans le document EP-A-0 173 109.

A titre de terpolymères pouvant être utilisés selon l'invention, on peut citer le produit de réaction de l'acide méthacrylique comme composant (a), de l'acrylate d'éthyle comme composant (b) et d'un macromonomère uréthane non-ionique comme composant (c), ayant la structure (II) suivante : 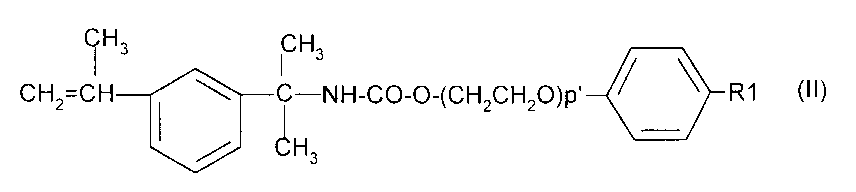 dans laquelle p' va de 6 à 150 et est égal de préférence à 30 et R1 est choisi parmi les radicaux alkyle comportant de 8 à 13 atomes de carbone, tel que décrit dans l'exemple 3 du document EP-A-0 173 109.

Le terpolymère acrylique préféré utilisé selon l'invention est obtenu à partir d'acide méthacrylique comme composant (a), d'acrylate de méthyle comme composant (b) et d'un macromonomère uréthane non-ionique comme composant (c), ayant la structure (III) suivante : 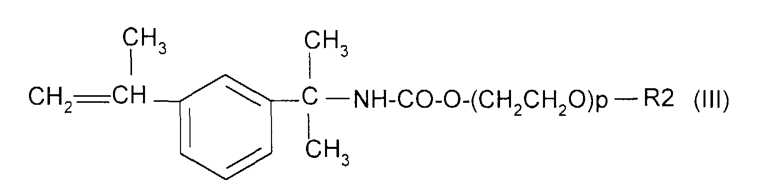 dans laquelle p va de 6 à 150 et R2 est choisi parmi les radicaux alkyle linéaires comportant de 18 à 26 et de préférence de 20 à 24 atomes de carbone. De préférence, le radical R2 dans la composé de formule (III) est un radical d'origine végétale, tel que le radical béhényle.

Les terpolymères utilisés selon l'invention sont généralement en dispersion aqueuse.

On peut utiliser notamment comme terpolymère, le terpolymère obtenu à partir d'acide acrylique, d'acrylate de méthyle et du composé de formule (III) où p est 40 et R2 est le radical béhényle. C'est le terpolymère acide méthacrylique/acrylate de méthyle/diméthyl m-isopropénylbenzylisocyanate d'alcool béhénylique éthoxylé à 40 OE, c'est-à-dire comportant 40 groupes oxyéthylénés.

Le terpolymère acrylique est présent dans la composition de l'invention dans des concentrations en matière active pouvant varier selon la concentration en huile et selon la viscosité souhaitée. La concentration en matière active de terpolymère va de préférence de 0,01 à 3 % en poids, mieux de 0,05 à 1 % en poids et préférentiellement de 0,2 à 0,5 % en poids par rapport au poids total de la composition. Pour la préparation d'émulsions très fluides, la quantité de terpolymère en matière active va de préférence de 0,05 à 1 % et mieux de 0,15 à 0,5 % en poids par rapport au poids total de la composition, et pour l'obtention de compositions riches en huiles, c'est à dire contenant au moins 40 % d'huiles, la quantité de terpolymère en matière active va de préférence de 0,4 à 1 % et mieux de 0,5 à 0,7 % en poids par rapport au poids total de la composition.

Dans la composition selon l'invention, le rapport terpolymère/phase huileuse va de 1/50 à 1/125. Pour la préparation d'émulsions très fluides, ce rapport va de préférence de 1/50 à 1/100 et mieux de 1/60 à 1/100. Quand la quantité de phase huileuse est importante (au moins 40 % en poids), ce rapport va de préférence de 1/75 à 1/125.

Selon un mode particulier de réalisation de l'invention, la composition de l'invention ne contient pas d'autres polymères que le terpolymère.

La phase huileuse de la composition selon l'invention représente généralement de 1 à 80 % et de préférence de 10 à 60 % en poids par rapport au poids total de la composition.

Pour la préparation d'émulsions très fluides, la quantité de phase huileuse va de préférence de 1 à 30 % et mieux de 10 à 25 % en poids par rapport au poids total de la composition, et pour l'obtention de compositions riches en huiles, la quantité de phase huileuse est de préférence supérieure à 40 % et va généralement de 40 à 80 % et mieux de 50 à 60 % en poids par rapport au poids total de la composition.

La nature de la phase huileuse de l'émulsion selon l'invention n'est pas critique. La phase huileuse peut ainsi être constituée par tous les corps gras et notamment les huiles, classiquement utilisés dans les domaines cosmétique ou dermatologique.

Parmi les huiles utilisables dans l'émulsion de l'invention, on peut citer par exemple les huiles végétales telles que les huiles de jojoba, avocat, amande douce, abricot, maïs et la fraction liquide de beurre de karité ; les huiles minérales comme l'huile de vaseline ; les huiles de synthèse comme le palmitate d'éthyl-2 hexyle, le myristate d'isopropyle, l'isoparaffine hydrogénée, l'isononanoate d'isononyle, l'octanoate de cétéaryle ; les huiles de silicone volatiles ou non volatiles ; et les huiles fluorées. Les autres corps gras susceptibles d'être présents dans la phase huileuse peuvent être par exemple les acides gras, les alcools gras et les cires.

Selon un mode particulier de réalisation de l'invention, la composition de l'invention contient au moins une huile de silicone, de préférence une huile de silicone volatile qui peut être choisie par exemple parmi les polydiméthylsiloxanes cycliques ou linéaires, et leurs mélanges. Les polydiméthylsiloxanes cycliques ou cyclométhicones comportent d'environ 3 à 9 atomes de silicium, et de préférence de 4 à 6 atomes de silicium et peuvent être par exemple le cyclohexadiméthylsiloxane et le cyclopentadiméthylsiloxane. Les polydiméthylsiloxanes linéaires volatiles comportent de préférence d'environ 3 à 9 atomes de silicium. Les polydiméthylsiloxanes linéaires volatiles ont généralement une viscosité à 25°C inférieure ou égale à 5 cSt tandis que les cyclométhicones ont généralement une viscosité à 25°C inférieure ou égale à 10 cSt.

La composition selon l'invention contient un milieu physiologiquement acceptable, c'est-à-dire compatible avec la peau, les lèvres, le cuir chevelu, les yeux et/ou les cheveux.

De façon connue, les compositions de l'invention peuvent contenir des adjuvants habituels dans les domaines considérés, tels que des actifs hydrophiles ou lipophiles, des conservateurs, des antioxydants, des parfums, des solvants, des charges (kaolin), des filtres, des matières colorantes, des agents basiques (triéthanolamine, soude) ou acides et encore des vésicules lipidiques. Ces adjuvants sont utilisés dans les proportions habituelles dans le domaine cosmétique, et par exemple de 0,01 à 30 % du poids total de l'émulsion, et ils sont, selon leur nature, introduits dans la phase aqueuse ou dans la phase huileuse de l'émulsion, ou encore dans des vésicules. Ces adjuvants ainsi que leurs concentrations doivent être tels qu'ils ne modifient pas la propriété recherchée pour l'émulsion de l'invention.

Comme solvants, on peut citer par exemple les mono-alcools linéaires ou ramifiés ayant de 1 à 8 atomes de carbone comme l'éthanol, le propanol, le butanol, l'isopropanol, l'isobutanol ; les polyéthylène glycols ayant de 6 à 80 oxydes d'éthylène ; les polyols comme le propylène glycol, l'isoprène glycol et le butylène glycol.

Comme actifs, on peut citer par exemple les hydratants tels que les polyols comme la glycérine et le sorbitol ; les agents kératolytiques ; les dépigmentants ; les amincissants, et tout actif approprié pour le but final de la composition.

Selon un mode particulier de l'invention, la composition de l'invention peut être exempte de nanopigments d'oxyde métallique.

La composition présente de préférence un pH qui respecte la peau et qui est compatible avec les terpolymères utilisés. Le pH de la composition va généralement de 6,5 à 8 et de préférence de 7 à 7,5.

Ainsi qu'indiqué ci-dessus, les compositions selon l'invention peuvent être plus ou moins fluides et elles peuvent donc se présenter sous forme de sérum, de lait, de crème ou de pâte. Ces compositions sont préparées selon les méthodes usuelles.

Les compositions, objets de l'invention, trouvent leur application notamment dans un grand nombre de traitements cosmétiques de la peau, des lèvres et des cheveux, y compris le cuir chevelu, notamment pour le traitement, la protection, le soin, le démaquillage et/ou le nettoyage de la peau, des lèvres et/ou des cheveux, et/ou pour le maquillage de la peau et/ou des lèvres. Elles peuvent être destinées aussi au traitement des peaux sèches et/ou des lèvres sèches.

Les compositions selon l'invention peuvent par exemple être utilisées comme produits de soin, de démaquillage et/ou de nettoyage pour le visage sous forme de crèmes ou de laits ou comme produits de maquillage (peau et lèvres) par incorporation de charges ou de colorants.

Aussi, l'invention a encore pour objet l'utilisation cosmétique de la composition telle que définie ci-dessus pour le traitement, la protection, le soin, le démaquillage et/ou le nettoyage de la peau, des lèvres et/ou des cheveux, et/ou pour le maquillage de la peau et/ou des lèvres.

L'invention a aussi pour objet l'utilisation de la composition telle que définie ci-dessus pour la fabrication d'une composition destinée au soin des peaux sèches et/ou des lèvres sèches.

L'invention a aussi pour objet un procédé de traitement cosmétique de la peau, y compris du cuir chevelu, des cheveux, et/ou des lèvres, caractérisé par le fait qu'on applique sur la peau, les cheveux et/ou les lèvres, une composition telle que définie ci-dessus.

Les exemples qui suivent permettront de mieux comprendre l'invention, sans toutefois présenter un caractère limitatif. Les quantités indiquées sont en % en poids, sauf mention contraire.

### Exemple 1 : Lait de soin pour le corps et le visage

| *Phase A* | |
|---|---|
| Terpolymère acide méthacrylique/acrylate de méthyle/ diméthyl m-isopropénylbenzylisocyanate d'alcool béhénylique éthoxylé à 40 OE, en dispersion aqueuse à 24 % (soit 0,5 % de matière active) | 2,1 % |
| Soude | 0,09 % |
| Conservateurs | 0,15 % |
| Eau déminéralisée qsp | 100 % |

| *Phase B* | |
|---|---|
| Cyclométhicone (cyclopentasiloxane) | 15 % |
| Huile d'amande douce | 15 % |

### Mode opératoire :

On ajoute la soude au polymère sous agitation et on réalise l'émulsion en versant la phase B dans la phase A sous agitation. On effectue ensuite une homogénéisation sous pression (500 bars).

On obtient une émulsion fluide particulièrement adaptée comme produit hydratant. Le toucher est très frais et non gras.

### Exemple 2 : Emulsion démaquillante

| *Phase A* | |
|---|---|
| Terpolymère acide méthacrylique/acrylate de méthyle/diméthyl m-isopropénylbenzylisocyanate d'alcool béhénylique éthoxylé à 40 OE, en dispersion aqueuse à 24 % (soit 0,3 % de matière active) | 1,25 % |
| Soude | 0,06 % |
| Conservateurs | 0,2 % |
| Isoprène glycol | 5 % |
| Eau déminéralisée qsp | 100 % |

| *Phase B* | |
|---|---|
| Palmitate d'éthyl-2 hexyle | 20 % |
| Huile de vaseline | 7 % |

Le mode opératoire est identique à celui de l'exemple 1.

On obtient une émulsion adaptée pour le démaquillage du visage et des yeux. Sa viscosité est adaptée pour le conditionnement de type flacon car elle a un aspect gélifié au repos mais s'écoule très facilement dès que l'on secoue le flacon. Sa viscosité est de 0,25 Pa.s (2,5 poises) au Rhéomat 180, mobile 2.

### Exemple 3 : Crème hydratante de nuit pour le visage

| *Phase A* | |
|---|---|
| Terpolymère acide méthacrylique/acrylate de méthyle/diméthyl m-isopropénylbenzylisocyanate d'alcool béhénylique éthoxylé à 40 OE, en dispersion aqueuse à 24 % (soit 0,4 % de matière active) | 1,68 % |
| Soude | 0,07 % |
| Conservateurs | 0,1 % |
| Glycérine | 7 % |
| Eau déminéralisée qsp | 100 % |

| *Phase B* | |
|---|---|
| Fraction liquide de beurre de karité | 15 % |
| Cyclométhicone (cyclopentasiloxane) | 10 % |
| Isoparaffine hydrogénée | 15 % |

Le mode opératoire est identique à celui de l'exemple 1.

On obtient une crème hydratante très confortable, même sur les peaux normales à tendance grasse malgré le fort pourcentage en huiles.

### Exemple 4 : Crème nutritive et hydratante pour peaux sèches

| Phase A | |
|---|---|
| Terpolymère acide méthacrylique/acrylate de méthyle/diméthyl m-isopropénylbenzylisocyanate d'alcool béhénylique éthoxylé à 40 OE, en dispersion aqueuse à 24 % (soit 0,5 % de matière active) | 2,1 % |
| Soude | 0,09 % |
| Conservateurs | 0,2 % |
| Glycérine | 2 % |
| Eau déminéralisée qsp | 100 % |

| *Phase B* | |
|---|---|
| Huile de vaseline | 15 % |
| Fraction liquide de beurre de karité | 20 % |
| Cyclométhicone (cyclopentasiloxane) | 15 % |
| Parfum | 0,4 % |

Le mode opératoire est identique à celui de l'exemple 1.

On obtient une émulsion particulièrement adaptée pour la réalisation de crèmes de nuit. Le toucher n'est pas gras malgré le fort pourcentage en huile.

### Exemple 5 : Crème démaquillante

| *Phase A* | |
|---|---|
| Terpolymère acide méthacrylique/acrylate de méthyle/diméthyl m-isopropénylbenzylisocyanate d'alcool béhénylique éthoxylé à 40 OE, en dispersion aqueuse à 24 % (soit 0,5 % de matière active) | 2,1 % |
| Soude | 0,09 % |
| Conservateurs | 0,2 % |
| Glycérine | 2 % |
| Eau déminéralisée qsp | 100 % |

| *Phase B* | |
|---|---|
| Huile de vaseline | 25 % |
| Huile de silicone (Dimethicone 10 cSt) | 15 % |
| Isononanoate d'isononyle | 20 % |

Le mode opératoire est identique à celui de l'exemple 1.

On obtient une crème particulièrement efficace pour le démaquillage des mascaras "waterproof" et des produits sans transferts. Elle est éliminée soit par rinçage soit par simple essuyage.

### Exemples comparatifs 1 et 2 :

On a remplacé dans la composition de l'exemple 5, le terpolymère utilisé selon l'invention par un polyacrylamide (Hostacerin AMPS ; nom CTFA : Ammonium polyacryldimethyltauramide) (exemple comparatif 1) ou du Pemulen TR2 (exemple comparatif 2). On a obtenu les résultats suivants :

| Composition | Exemple 5 | Exemple comparatif 1 | Exemple comparatif 2 |
|---|---|---|---|
| pH à la température ambiante (20-25°C) | 7,05 | 7,25 | 6,7 |
| Stabilité | Emulsion très fine Texture épaisse | Emulsion grossière* Texture très fluide | Emulsion grossière* Texture assez fluide |
| | | | |
| | Stable à toutes températures | Déstabilisée après 4 semaines à 45°C | Relargage d'eau en surface après 2 mois à 20-25°C |
| Viscosité à T0 à environ 25°C au Rhéomat 180 | 4,7 Pa.s (mobile 4) | 0,131 Pa.s (mobile 2) | 0,9 Pa.s (mobile 3) |

| | | | |
|---|---|---|---|
| *une émulsion grossière est une émulsion comportant de gros globules huileux, c'est-à-dire ayant une dimension moyenne supérieure à 20 microns. | | | |

Il ressort du tableau ci-dessus que le polyacrylamide et le Pemulen TR2 ne permettent pas d'obtenir ces textures crèmes épaisses comportant un fort taux d'huile qui soient stables au pH proche de la neutralité.

### Exemple 6 : Masque

| *Phase A* | |
|---|---|
| Terpolymère acide méthacrylique/acrylate de méthyle/diméthyl m-isopropénylbenzylisocyanate d'alcool béhénylique éthoxylé à 40 OE, en dispersion aqueuse à 24 % (soit 0,4 % de matière active) | 1,67 % |
| Soude | 0,07 % |
| Kaolin | 15 % |
| Conservateurs | 0,3 % |
| Glycérine | 10 % |
| Eau déminéralisée qsp | 100 % |

| *Phase B* | |
|---|---|
| Huile de maïs | 15 % |
| Cyclométhicone (cyclopentasiloxane) | 10 % |
| Octanoate de cétéaryle | 10 % |

Le mode opératoire est identique à celui de l'exemple 1.

On obtient un masque particulièrement bien toléré par les peaux sensibles. Il se rince ou s'essuie après quelques minutes de temps de pose.

## Revendications

1. Composition sous forme d'émulsion huile-dans-eau comprenant dans un milieu physiologiquement acceptable, une phase huileuse dispersée dans une phase aqueuse, caractérisée par le fait qu'elle contient au moins un terpolymère acrylique obtenu à partir de (a) un acide carboxylique à insaturation α,β-éthylénique, (b) un monomère à insaturation éthylénique non tensioactif différent de (a), et (c) un monomère uréthanne non-ionique qui est le produit de réaction d'un composé amphiphile non-ionique monohydrique avec un isocyanate à insaturation monoéthylénique,
le rapport en poids terpolymère/phase huileuse allant de 1/50 à 1/125.

2. Composition selon la revendication 1, caractérisée en ce qu'elle est exempte de tensioactif.

3. Composition selon la revendication 1 ou 2, caractérisée par le fait que le terpolymère acrylique comprend, par rapport au poids total du terpolymère, :
(a) environ de 20 à 70 % en poids, et de préférence de 25 à 55 % en poids, d'un acide carboxylique à insaturation α,β-éthylénique,
(b) environ de 20 à 80 % en poids, et de préférence de 30 à 65 % en poids, d'un monomère à insaturation éthylénique non tensioactif diifférent de (a), et
(c) environ de 0,5 à 60 % en poids, et de préférence de 10 à 50 % en poids, d'un monomère uréthanne non-ionique qui est le produit de réaction d'un composé amphiphile non-ionique monohydrique avec un isocyanate à insaturation monoéthylénique.

4. Composition selon l'une quelconque des revendications précédentes, caractérisée par le fait que l'acide carboxylique à insaturation α,β-monoéthylénique (a) est choisi parmi l'acide acrylique, l'acide méthacrylique, l'acide crotonique, l'acide itaconique et l'acide maléique.

5. Composition selon l'une quelconque des revendications précédentes, caractérisée par le fait que l'acide carboxylique à insaturation α,β-monoéthylénique (a) est l'acide méthacrylique.

6. Composition selon l'une quelconque des revendications précédentes, caractérisée en ce que le monomère à insaturation monoéthylénique non tensioactif (b) est choisi parmi les acrylates et méthacrylates d'alkyle en C₁-C₄, le styrène, le vinyltoluène, l'acétate de vinyle, l'acrylonitrile et le chlorure de vinylidène.

7. Composition selon l'une quelconque des revendications précédentes, caractérisée en ce que le monomère à insaturation monoéthylénique non tensioactif (b) est l'acrylate de méthyle ou l'acrylate d'éthyle.

8. Composition selon l'une quelconque des revendications précédentes, caractérisée en ce que le composé amphiphile non-ionique monohydrique utilisé pour obtenir le monomère uréthane non-ionique est un composé ayant la formule (I) suivante :
R-(OCH₂CHR')m-(OCH₂CH₂)n-OH (I)
dans laquelle R est choisi parmi les groupes alkyle comportant de 6 à 30 atomes de carbone et les groupes aralkyle comportant de 8 à 30 atomes de carbone, R' est choisi parmi les groupes alkyle comportant de 1 à 4 atomes de carbone, n est un nombre moyen allant d'environ 6 à 150 et m est un nombre moyen allant d'environ 0 à 50, à la condition que n soit au moins aussi grand que m et que n + m = 6 à 150.

9. Composition selon la revendication précédente, caractérisée en ce que, dans le composé de formule (I), R est choisi parmi les groupes alkyle comportant de 18 à 26 atomes de carbone et alkyl-(C₈-C₁₃)-phényle ; le groupe R' est le groupe méthyle ; m = 0 et n = 6 à 150.

10. Composition selon l'une quelconque des revendications précédentes, caractérisée par le fait que le monoisocyanate à insaturation éthylénique utilisé pour former le monomère uréthanne non-ionique (c) est l'α,α-diméthyl-m-isopropényl-benzylisocyanate.

11. Composition selon l'une quelconque des revendications précédentes, caractérisée par le fait que le terpolymère acrylique est obtenu par copolymérisation en dispersion aqueuse à partir de l'acide méthacrylique comme composant (a), de l'acrylate de méthyle comme composant (b) et d'un macromonomère uréthane non-ionique comme composant (c), ayant la structure (III) suivante : 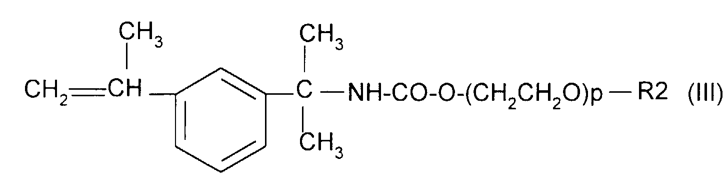 dans laquelle p va de 6 à 150 et R2 est choisi parmi les radicaux alkyle linéaires comportant de 18 à 26 atomes de carbone.

12. Composition selon l'une quelconque des revendications précédentes, caractérisée par le fait que le terpolymère est présent en une concentration en matière active allant de 0,01 à 3 % en poids par rapport au poids total de la composition.

13. Composition selon l'une quelconque des revendications précédentes, caractérisée par le fait que la phase huileuse représente de 1 à 80 % et de préférence de 10 à 60 % en poids par rapport au poids total de la composition.

14. Composition selon l'une quelconque des revendications précédentes, caractérisée en ce qu'elle contient au moins une huile de silicone volatile.

15. Composition selon l'une quelconque des revendications précédentes, caractérisée en ce qu'elle présente un pH allant de 6,5 à 8.

16. Utilisation cosmétique de la composition selon l'une quelconque des revendications 1 à 15, pour le traitement, la protection, le soin, le démaquillage et/ou le nettoyage de la peau, des lèvres et/ou des cheveux, et/ou pour le maquillage de la peau et/ou des lèvres.

17. Utilisation de la composition selon l'une quelconque des revendications 1 à 15, pour la fabrication d'une composition destinée au soin des peaux sèches et/ou des lèvres sèches.

18. Procédé de traitement cosmétique de la peau, des cheveux, et/ou des lèvres, caractérisé par le fait qu'on applique sur la peau, les cheveux et/ou les lèvres, une composition selon l'une quelconque des revendications 1 à 15.
